# EUROPEAN PATENT SPECIFICATION

(11) **EP 0 763 131 B1**
(45) Date of publication and mention of the grant of the patent: **25.08.1999**
(21) Application number: 95921033.7
(22) Date of filing: 02.06.1995
(51) Int. Cl.: C12Q 1/37, C07K 16/40, G01N 33/68, G01N 33/543, G01N 33/569

(54) **ASSAY OF TETANUS- AND BOTULINUM TOXINS**
BESTIMMUNG DER TETANUS- UND BOTULINUM TOXINE
DOSAGE DE TOXINES DU TETANOS ET DU BOTULISME

(30) Priority: 03.06.1994 GB 9411138
(43) Date of publication of application: 19.03.1997
(73) Proprietor: Microbiological Research Authority, Salisbury, Wiltshire SP4 0JG (GB)
(72) Inventor: SHONE, Clifford, Charles, Salisbury SP5 3BN (GB); HALLIS, Bassam, Salisbury, Wiltshire SP2 7BT (GB); JAMES, Benjamin, Arthur, Frederick, Salisbury, Wiltshire SP5 3TE (GB); QUINN, Conrad, Padraig, Salisbury, Wiltshire SP1 3QS (GB)
(74) Representative: Schlich, George William
(86) International application number: GB9501279
(87) International publication number: WO9533850

(56) References cited:
- EP-A- 0 529 719
- US-A- 5 171 662
- EUROPEAN JOURNAL OF BIOCHEMISTRY, vol. 225, no. 1, October 1994 pages 263-270, C .C SHONE ET AL. 'Peptide substrate specificity and properties of the zinc-endopeptidase activity of botulinum type B neurotoxin.'
- BIOCHEMISTRY, vol. 33, 27 December 1994 pages 15365-15374, P. FORAN ET AL. 'Differences in the protease activities of Tetanus and Botulinum B toxins revealed by the cleavage of vesicle-associated membrane protein and various sized fragments.'
- NATURE, vol. 372, no. 1, 1 December 1994 pages 415-416, O. ROSSETTO ET AL. 'SNARE motif and neurotoxins.'
- J.Cell.Biol., vol.109, p.3039-3052, (1989)

## Description

This invention relates to assays for toxins, in particular botulinum neurotoxins and tetanus toxins, that have peptidase activity. The invention also relates to antibodies useful in the assays and to solid-phase peptides useful in the assays.

The botulinum neurotoxins are a family of structurally similar, but antigenically different protein neurotoxins which act on the peripheral nervous system to block neuromuscular transmission. These neurotoxins are extremely potent, with a human lethal dose in the order of micrograms, and give rise to the rare but frequently fatal disease, botulism. Assays for the botulinum neurotoxins are currently used in both the food and pharmaceutical industry. The food industry employs assays for the botulinum neurotoxins to validate new food packaging methods and to ensure food safety. With the growing clinical use of the botulinum toxins, the pharmaceutical industry requires accurate assays for these toxins for both product formulation and quality control.

It is known to assay for botulinum toxin in foodstuffs using the mouse lethality test. This test has been the industry standard for many years, though over the past 10 years a number of immunoassay methods have been developed in an attempt to replace the mouse test in the majority of applications.

One such assay operates by addition of a test sample to a plate or column to which is attached an antibody that binds to toxin present in the sample. A further antibody is typically used to detect bound toxin. These enzyme-linked immunoassays (ELISA) have the advantages that they are specific to one botulinum toxin type and can be performed rapidly, in less than 2 hours. The ELISAs, however, suffer from several drawbacks:-
(a) They do not measure the biological activity of the toxins,
(b) They cannot distinguish between active and inactive toxin, and
(c) Due to antigenic variations some toxins are not detected by these assays which therefore give rise to false negatives.

The botulinum neurotoxins have recently been shown to possess highly specific zinc-endopeptidase activities within their light sub-units. Depending on the neurotoxin type these act to cleave small proteins within the nerve cell which are involved in neurotransmitter release. Botulinum types A and E toxins cleave protein SNAP-25. Botulinum types B, D, F and G and tetanus toxins cleave vesicle-associated membrane protein (VAMP-also called synaptobrevin). Botulinum type C toxin cleaves the protein syntaxin.

In the development of further toxin assays, various procedures have been devised for the evaluation of endopeptidase activities. Liquid chromatography procedures are known and are based on resolution of the peptide product and subsequent evaluation. These procedures are time-consuming, expensive and do not lend themselves readily to automation. It is also known to use spectrophotometric methods, requiring the development of suitable chromogenic peptide reagents. Such methods provide a continuous precise assay for endopeptidases. Spectrophotometric methods, however, require relatively pure preparations of enzyme and are not normally suitable for evaluation of endopeptidase activities in crude or particulate samples.

Despite these efforts, at present, the only convenient assay for the biological activity of the botulinum neurotoxins, and the only assay that is FDA approved, remains the mouse lethality test. This test suffers from a number of drawbacks:-
(a) It is expensive and uses large numbers of laboratory animals,
(b) It is non-specific unless performed in parallel with toxin neutralization tests using specific anti-sera, and
(c) It is not very accurate unless large animal groups are used.

The present invention describes a novel assay system for toxins, using novel reagents. The assay aims to overcome or at least mitigate many of the drawbacks of present *in vitro* assays for these toxins.

Accordingly, a first aspect of the invention provides a toxin assay comprising the steps:-
(a) combining a test compound with a substrate and with antibody, wherein the substrate has a cleavage site for the toxin and when cleaved by toxin forms a product, and wherein the antibody binds to the product but not to the substrate; and
(b) testing for the presence of antibody bound to the product wherein,
   (i) product if present is attached to a solid phase assay component and binds the antibody; and
   (ii) substrate if present is similarly attached to a solid phase assay component.

This has the advantage that the assay is capable of distinguishing between active and inactive toxin - as inactive toxin will have reduced or no activity. Also, antigenic variation between toxins of the same group will not significantly affect the working of the assay, because it is toxin activity (ability to cleave the substrate) that is being measured and not the precise antigenic make-up of the toxin.

In use of an embodiment of the invention, cleavage of the substrate by toxin present in the test compound generates a product that is recognised by and binds to the antibodies of the assay, which antibodies do not bind to the uncleaved substrate. It is preferred that the toxin cleaves the substrate at a single location so as to generate two products, one of which binds to the antibodies.

In another embodiment of the invention, the substrate is a peptide or protein that contains a cleavage site for the toxin and cleavage of the substrate generates new peptides having N- and C- terminal ends. The antibody binds to one of these newly formed peptides. A particular assay of the invention is for assay of a botulinum toxin or tetanus toxin and the assay determines whether amounts of one of these toxins are present in the test compound. In this particular assay, the substrate of the invention is a peptide or a protein that is cleaved by endopeptidase activity of the botulinum toxin or tetanus toxin. An advantage of the assay is that it distinguishes between active and inactive toxin, as the assay is a measurement of the peptidase activity present in the sample.

A further embodiment of the assay of the invention comprises:-
(i) combining the test compound with a solid phase comprising a peptide selected from VAMP, SNAP-25, syntaxin and fragments thereof,
(ii) washing the test compound from the solid phase,
(iii) combining the solid phase with an antibody adapted for binding selectively with peptide cleaved by toxin, and
(iv) detecting conjugates of the antibody with cleaved peptide.

Preferably, the antibody is adapted to bind selectively to a peptide selected from SEQ ID NO:s 1-7.

Another embodiment of the assay comprises:-
(i) adding a test solution to an assay plate comprising immobilized peptide, the peptide being selected from VAMP, SNAP-25, syntaxin and fragments thereof,
(ii) incubating the assay plate,
(iii) washing the plate with a buffer,
(iv) adding to the plate an antibody solution, said solution comprising an antibody adapted selectively to bind to a peptide selected from (1) a peptide the C-terminal end of which is selected from SEQ ID NO:s 1, 3 and 5, and (2) a peptide the N-terminal end of which is selected from SEQ ID NO:s 2, 4 and 6,
(v) incubating the assay plate,
(vi) washing the plate with a buffer, and
(vii) measuring the presence of antibody on the assay plate.

In steps (ii) and (v) a suitable incubation period is from twenty minutes to 3 hours, at 35-39°C.

In use, the antibody can be linked to an enzyme and antibody on the plate measured by adding a substrate and observing conversion into a detectable, e.g. coloured, product.

It has already been noted that the assay of the invention detects active toxin in a compound under investigation. Some toxins, notably botulinum toxins, exist also in an inactive conformation, and as such would not be identified by the assay. A further embodiment of the assay includes pre-treatment of the test compound, to convert inactive toxin into active toxin. This is achieved for example by a protease, such as trypsin.

It is further preferred that the assay incorporates a substrate attached to a solid phase, for example covalently linked, such as via a terminal cysteine residue, to a solid phase of the assay.

The assay procedure of the invention thus typically represents a solid-phase microtitre based assay which can be applied to a number of specific toxins such as the botulinum neurotoxins and tetanus toxin. In contrast to spectrophotometric assays, the procedure of the invention can be easily automated and can be applied to very crude or particulate preparations of toxin endopeptidases. The procedure is also relatively inexpensive and easy to use.

For detection of antibodies bound to the cleaved substrate it is optional for the antibodies to be linked to a marker molecule or an enzyme. In an example, the marker is horse radish peroxidase which can be detected using conventional and well-known techniques.

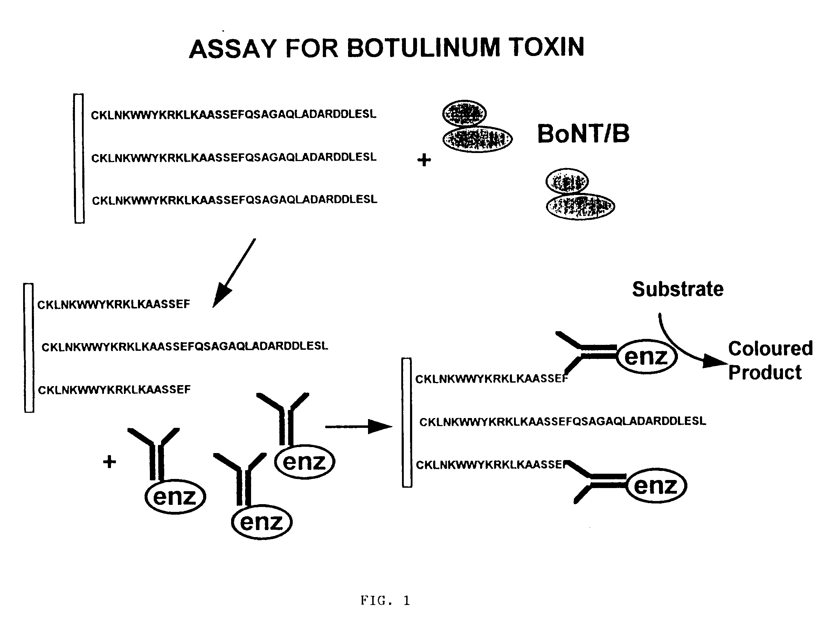

In a particular embodiment of the invention, synthetic peptide substrates for the botulinum toxins (derived from sequences of intracellular proteins that are targets for the toxins) are prepared and used as the solid-phase endopeptidase substrate component in an assay system that follows the procedure:-
Step 1 - test solutions containing botulinum toxin are incubated with the solid - phase endopeptidase substrate. This results in the cleavage of the peptide at a specific point in the sequence which is dependant on the toxin type (this is illustrated in fig. 1).
Step 2 - incubation with antipeptide antibody reagent. This antibody reagent is specific to one of the newly cleaved N- or C- terminal ends of the solid-phase peptide and does not recognise the intact peptide. The antibody reagent is covalently conjugated to an enzyme marker, such as peroxidase or alkaline phosphatase. It is a further option to have present two separate antibodies that bind, respectively, one to the N-terminal ends and the other to the C-terminal ends of the newly cleaved peptides.
Step 3 - incubation with substrate for the enzyme marker or further amplification by commercially available techniques.

It is seen that the assay of this latter embodiment has two main components.

The first component is an endopeptidase substrate component which contains or consists of a protein or peptide that is cleaved by the toxin endopeptidase to be assayed. This will typically be incorporated into the solid-phase component of the assay.

The endopeptidase substrate component may be, for example, the protein target of the endopeptidase (such as syntaxin, VAMP or SNAP-25) or a fragment of these proteins that is cleaved by the toxin endopeptidase. The endopeptidase substrate component may alternatively be a fusion protein comprising the above linked to another protein.

The solid-phase may be an ELISA plate, bead, dip-stick or any other matrix that can be used for immobilizing the endopeptidase substrate component. The solid-phase component may also be a nitrocellulose membrane or the equivalent as used in the Western blotting technique.

The second component is the antibody component which is an antibody raised against a short polypeptide (typically 6-8 amino acid residues in length) representing the sequence of either the newly generated N- or the newly generated C-terminal peptide obtained after cleavage of the endopeptidase substrate component by the toxin endopeptidase. The antibody has the properties that it does not recognise the endopeptidase substrate component but instead recognise only the cleaved peptide product.

The antibody may be linked to an enzyme, or a radioactive or fluorescent marker. Alternatively, the antibody may be the free protein and a second anti-antibody used in the assay procedure. The antibody may further be polyclonal or monoclonal in make up, an antibody fragment, or an antibody or antibody-fragment fused or linked to another protein.

The antibody for use in the assay of the first aspect characterised by the following properties:-
(a) the antibody binds to composition consisting of a peptide that is the product of cleavage of a substrate by a toxin endopeptidase, attached to a solid phase via a cysteine residue, and
(b) the antibody does not bind to the uncleaved substrate.

The antibody does not bind to the substrate, which is linked covalently or otherwise to a carrier molecule. It is preferred that the substrate is a peptide, more preferably a nerve cell peptide, that is cleavable by a toxin according to the first aspect of the invention. The peptide is preferably selected from syntaxin, VAMP and SNAP-25.

It is further preferred that the peptide substrate is cleaved by the toxin endopeptidase at a limited number of locations, and preferably at two, or more preferably one, location so as to generate a limited number of cleavage products, and that the antibody binds selectively to one of these products.

In preferred embodiments the antibody recognises one of the following peptide sequences:-

| | | |
|---|---|---|
| 1. | KAASSEF-n terminal | (SEQ ID NO:1) |
| 2. | LQAGASQ-c terminal | (SEQ ID NO:2) |
| 3. | RIDEANQ-c terminal | (SEQ ID NO:3) |
| 4. | GLMKTAR-n terminal | (SEQ ID NO:4) |
| 5. | QNRQIDR-c terminal | (SEQ ID NO:5) |
| 6. | SDAKEMI-n terminal. | (SEQ ID NO:6) |
| 7. | KAASTEF-n terminal. | (SEQ ID NO:7) |

In addition, the preferred antibodies do not recognise the one of the sequences 1-7 immediately above when that sequence is part of an intact, uncleaved peptide and that sequence does not contain the free N- or C-terminal ends shown immediately above.

An antibody for use in a toxin assay is prepared, for example, by the procedure:-
- identifying a peptide that is cleaved by the toxin to generate a cleavage product,
- immunizing an animal, such as a rabbit, with the cleavage product,
- isolating antibodies to the cleavage product from the animal, and
- checking that the antibodies do not cross react with the peptide.

The antibody for use in a toxin assay may be obtained by a method comprising: Identifying a macromolecule that is cleaved by a toxin into cleavage products, immunizing an animal against one of the cleavage products, isolating antibodies that bind to said one of the cleavage products and checking the antibody does not cross-react with the macromolecule. An embodiment of this method comprises:-
(i) identifying a substrate that is cleaved by a toxin, thereby forming cleavage products,
(ii) administering to an animal an immunogen comprising a carrier protein linked to one of the cleavage products or to a fragment of one of the cleavage products so as to raise immunogen specific antibodies.
(iii) isolating from the animal an immunogen-specific antibody, and
(iv) checking the antibody does not cross-react with the substrate.

The macromolecule is preferably a peptide or a protein. It is particularly preferred that the peptide or protein is cleaved into two fragments.

The method enables production of antibodies that possess a surprising and advantageous property, in particular for performing assays for toxin. The property of interest is the binding affinity for the shortened cleavage product, enabling binding to this product, uncontaminated by an affinity of any significance to the intact, uncleaved macromolecule.

In another embodiment, the method comprises:-
(i) identifying a peptide or protein that is cleaved by a toxin, thereby forming at least first and second cleavage products,
(ii) immunizing an animal with an antigen selected from (1) a terminal fragment of the first cleavage product, (2) a synthetic fragment of the first cleavage product, (3) an analogue of (1) and (4) a carrier molecule linked to any of (1)-(3),
(iii) isolating from the animal an antibody that binds to the first cleavage product.

Many carrier molecules are known in the art, and preferred carriers include keyhole limpet hemocyanin, bovine serum albumin and ovalbumin.

In a further aspect the invention provides a component of a toxin assay which is a peptide substrate attached to a solid phase of a toxin assay, the peptide substrate being a target for cleavage by toxin endopeptidase. Preferably, the peptide is selected from the intact peptide and fragments of:-
1. VAMP
2. SNAP-25
3. Syntaxin.

Particularly preferred target peptide sequences for use in the assay are selected from SEQ ID NOs. 8-11 illustrated below.

Further preferred peptides are peptides that include a sequence selected from SEQ ID NO:s 1-7.

The solid-phase peptides of the assay are stable and suitable for incorporation into test kits that provide for convenient and efficient toxin assays.

The assay procedures thus encompass various features:-
(i) The production of an antibody to a polypeplide sequence (e.g. XXXXN) which is recognised only when amino acid N is free and not when the same sequence makes up a longer peptide (e.g. XXXXNXXXX). It is surprising that suitable antibodies can be raised that show non-reactivity to the longer peptide.
(ii) The use of the properties of such an antibody in a rapid, solid-phase assay for toxin endopeptidase. In the example of the botulinum or tetanus neurotoxin assay, the procedure exploits the zinc-endopeptidase activities of these toxins.
(iii) An assay that uses toxin target molecules, such as nerve cell peptides, as solid-phase assay components.
(iv) An assay that uses the antibodies described in (i) as solid-phase assay components.

A feature of the invention is that antibodies used to detect cleaved peptide do not bind to uncleaved peptide; for, clearly, such binding would generate false positives and compromise assay accuracy. A convenient means of determining that antibodies do not cross-react with intact peptide is to screen antibodies using two plates. The first contains intact peptide attached to a solid phase. The second contains cleaved peptide attached to a solid phase, a free end of the cleaved peptide being, for example, one of SEQ ID NO:s 1-7 or another sequence recognised by assay antibodies.

Antibodies under screening are added to both plates, allowed to incubate for a period and the plates are then washed and subsequently tested for the presence of bound antibody. Suitable antibodies, i.e. non cross-reacting ones, generate a large signal on the second plate, indicating the presence of bound antibody and a weak signal, preferably virtually no signal, on the first plate.

If a number of potential assay antibodies are tested then it is straightforward to compare the ratio of signal (second plate result) to noise (first plate result) so that antibodies are selected with a high selectivity for cleaved peptide over intact. In an embodiment, this ratio is at least 10:1, preferably at least 20:1 and more preferably at least 50:1. A particularly preferred ratio would be 100:1 or higher, reflecting an affinity for cleaved peptide at least 2 orders of magnitude greater than affinity for intact peptide.

The invention is of advantage in that it provides an assay that measures biological activity of the toxins, e.g. botulinum neurotoxin, tetanus toxin, and with a sensitivity comparable to that of a conventional ELISA system. The inventors have observed that the assay is capable of detecting less than 1 ng of toxin per ml in the case of botulinum A and B toxins. For many workers, accurate quantification of toxin would be highly desirable for use in risk assessment. Also, false negatives are unlikely as the assay is a direct measurement of toxin endopeptidase activity. The observations by the inventors are that assay noise levels in serum and other samples are significantly lower than those obtainable in conventional immunoassay systems. The inventors have to date had no false negatives or false positives with the assay for botulinum B toxin and the noise levels were lower in toxin samples containing serum than the noise levels that can be obtained with a conventional ELISA. Further, the peptide solid-phase assay component is stable with a very long shelf life.

The assay and assay components of the invention described are particularly sensitive for toxin and have been found by the inventors to have comparable and improved sensitivity to ELISAs for toxin. A further advantage of the invention is that it has potential to be more sensitive, as further specific antibodies are identified for use in the assay. Contrast this situation with that for ELISAs, which rely on antibody affinity for toxin. As this affinity is already optimal due to extensive work in isolating high affinity antibodies, few increases in ELISA sensitivity are expected.

There now follows a number of examples of the invention illustrated by drawings in which:-
- Fig. 1: shows a schematic representation of a botulinum toxin assay;
- Figs. 2-4: show results achieved using the botulinum toxin assay of Example 1;
- Figs. 5-6: show results in which the botulinum toxin assay of Example 1 is further improved and made more sensitive by signal amplification and toxin activation procedures described in Example 2; and
- Fig. 7: shows results achieved using the botulinum toxin assay of Example 4.

### Example 1. An Assay for Botulinum Type B Neurotoxin (BoNT/B)

For the botulinum type B assay a peptide representing the sequence of VAMP isoform-1, residues 60-94 (SEQ ID NO:9, plus an C-terminal cysteine residue) was used:

Microtitre assay plates were prepared as follows:- The above peptide was diluted to a final concentration of 10 µgml⁻¹ in 0.05 M sodium phosphate buffer, pH 6.5 containing 1mM EDTA and added (100 µl/well) to a Sulphydryl Binding Plate (Costar). After incubation for 1 hour at room temperature the peptide solution was removed and the plates washed 3 times with phosphate buffered saline (PBS), pH 7.4 (no detergent is used at this stage in the washing). Any remaining binding sites on the plates were then blocked by the addition (100 µl/well) of a PBS buffer containing 0.1% Tween 20 and 5% Foetal bovine serum. The plates were then incubated for one hour at 37°C with continuous shaking after which the blocking solution was removed.

Assay of *Clostridium botulinum* type B neurotoxin:- Toxin solutions were diluted in an assay buffer (e.g. 0.05M Hepes buffer, pH 7.4 containing 10 µM ZnCl₂ and 1% foetal bovine serum) containing 10 mM dithiothreitol (or an equivalent reagent to provide reducing conditions for the endopeptidase activity of the toxins) and added (100 µl/well) to peptide coated microtitre plates. The plate is then incubated for one hour at 37°C with continuous shaking. After this period in which BoNT/B cleaves the peptide as depicted in Fig 1, the plates were washed 3 times with PBS containing 0.1% Tween 20®.

Antibody specific to the newly cleaved peptide is then added. In this example of the assay, the antibody is specific to the following cleaved sequence of VAMP (SEQ ID NO:1 plus C-terminal cysteine):-

The antibody (diluted in PBS buffer containing 0.1% Tween 20® and 5% foetal bovine serum) is added (100 µl/well) and incubated for one hour at 37°C with continuous shaking. The plates were then washed 3 times with PBS containing 0.1% Tween 20.

If the antibody is conjugated to peroxidase, appropriate peroxidase substrates are added at this stage to develop the colour and provide the results of the assay.

An alternative is that the antibody is in its free form, in which case the addition of a commercially available second antibody is required. If, for example, the anti-peptide antibody is raised in guinea-pigs, then this second antibody is an antiguinea-pig peroxidase conjugate. The second antibody conjugate (diluted in PBS buffer containing 0.1% Tween 20® and 5% foetal bovine serum) is added (100 µl/well) and incubated for one hour at 37°C with continuous shaking. After washing off the second antibody (x3 with PBS containing 0.1% Tween 20®), appropriate peroxidase substrates may be added at this stage to develop the colour and provide the results of the assay.

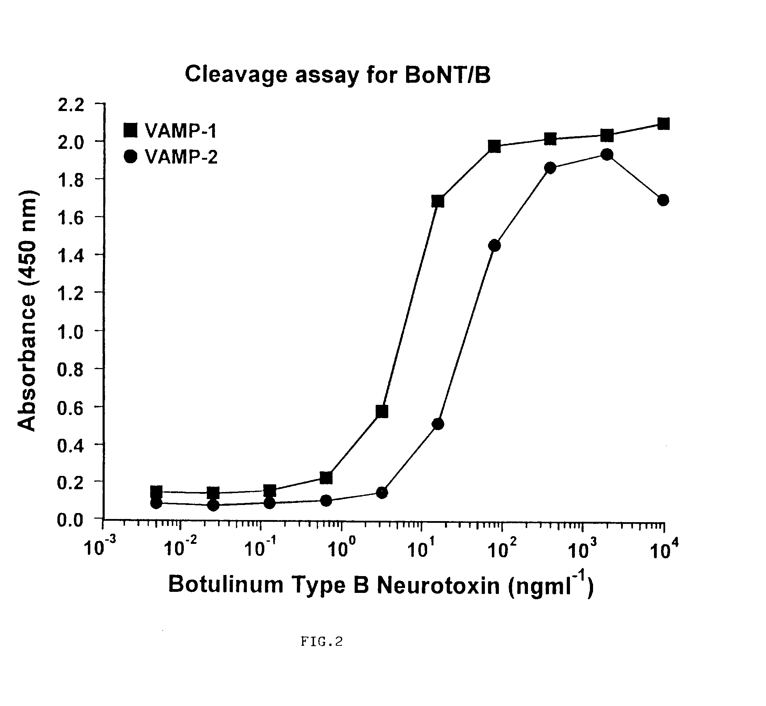

Figure 2 illustrates the results of a typical assay for BoNT/B using the two antibody detection system described above. The data illustrate the use of two different VAMP peptides as the solid-phase peptide in the assay. The sequences of these peptides near the cleavage site for BoNT/B represent the two isoforms of human VAMP.

The data show that, at an arbitrary cut-off point of 0.3 absorbance units above background, the sensitivity for the detection of BoNT/B in these assay was approximately 1ng/ml using the VAMP-1 peptides as the solid-phase and approximately 5ng/ml using the VAMP-2 peptides as the solid-phase.

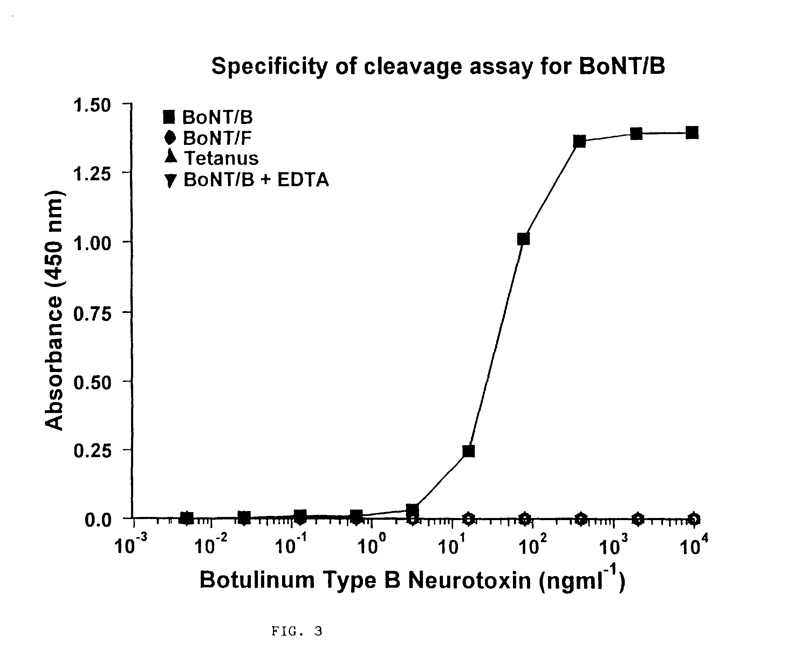

Figure 3 illustrates the specificity of the assay system for BoNT/B. Zero absorbance was recorded for (1) BoNT/F, (2) Tetanus toxin and (3) BoNT/B plus EDTA. These data show that an assay designed to detect BoNT/B did not give false-positive results in the presence of the closely related botulinum type F and tetanus toxins. The data also show that no signal was obtained in assays performed with BoNT/B in the presence of EDTA (which is a metal ion chelating agent). The latter result illustrates that the assay is dependent on the zinc-metalloprotease activity of BoNT/B.

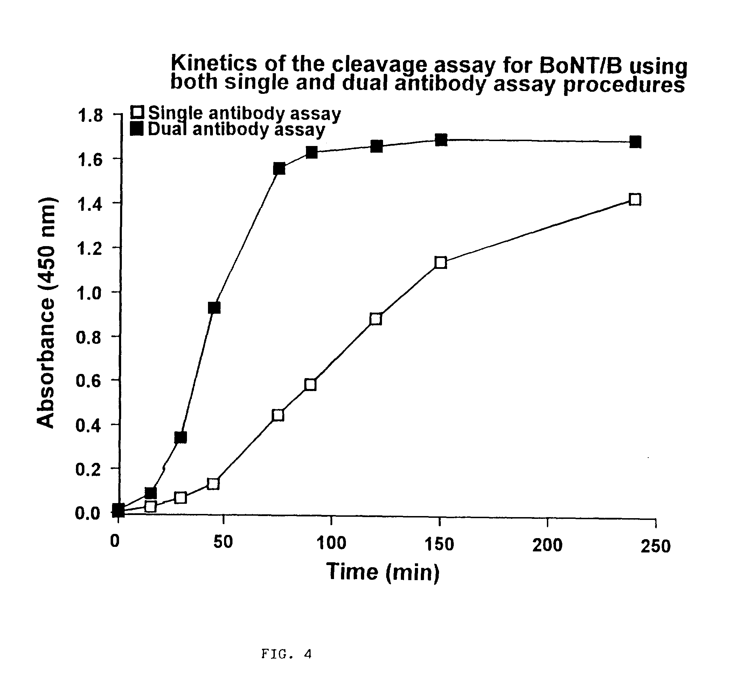

Figure 4 illustrates the assay kinetics of solid-phase peptide cleavage by BoNT/B using both the single antibody (i.e. antipeptide antibody directly conjugated to peroxidase) and dual antibody (i.e. in which the free antipeptide antibody is used in conjunction with a anti-species peroxidase conjugate) detection systems. The concentration of BoNT/B was fixed at 100ng/ml and the assay signal measured at various times. The data show that the dual antibody assay system provides the more rapid and sensitive means with which to measure solid-phase peptide cleavage by BoNT/B .

### Example 2. Amplification and Enhancement of Assays for the Botulinum Neurotoxins.

The are a number of ways in which the assays for the botulinum toxins such as that described in Example 1 may be further enhanced to provide greater sensitivity:-
(a) Further amplification of the assay signal using commercially available amplification systems such as the ELAST™ (Dupont) system.
(b) Limited trypsin treatment of the neurotoxin prior to assay. All of the botulinum neurotoxins are produced, in the bacterium, as single polypeptide chains which are then subsequently activated by specific bacterial proteases to give the active, dichain form of the toxin In some instances, notably in the case of botulinum neurotoxin types B and E, the toxins are not fully activated and can contain up to 100% inactive neurotoxin. These inactive, single chain, forms of the toxin express the endopeptidase activity of the toxin either weakly or not at all. Limited trypsin treatment of the botulinum neurotoxins converts the single chain toxin form to the di-chain, active toxin form. Thus, in botulinum neurotoxin types which contain a portion of single chain toxin, trypsin treatment results in both an increase in the endopeptidase activity and specific toxicity of the neurotoxin.

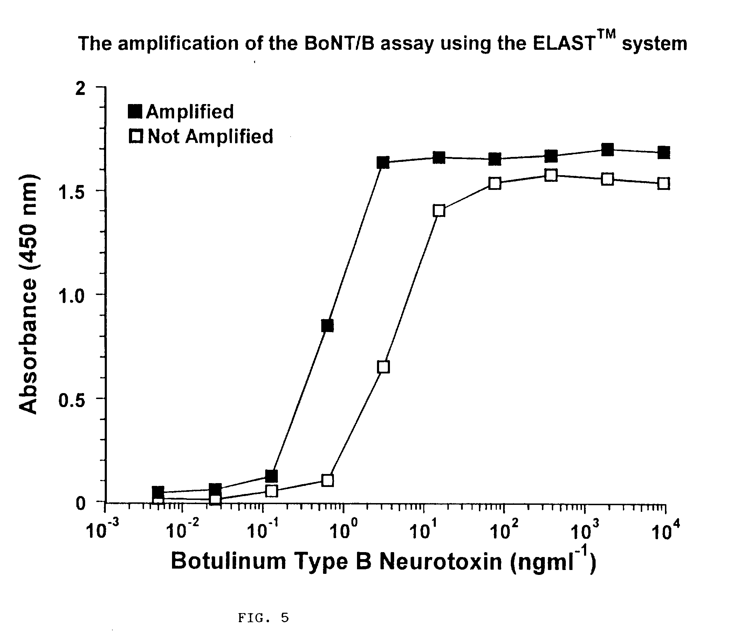

Figure 5 illustrates the increase in sensitivity of an assay for BoNT/B when an amplification system such as the ELAST system is applied to a typical assay as described in Example 1. The data show that, at an arbitrary cut-off point of 0.3 absorbance units above background, the sensitivity for the detection of BoNT/B in the non-amplified assay was approximately 1 ng/ml while that in the amplified assay system was almost 10-fold higher giving a sensitivity close to 0.1 ng/ml.

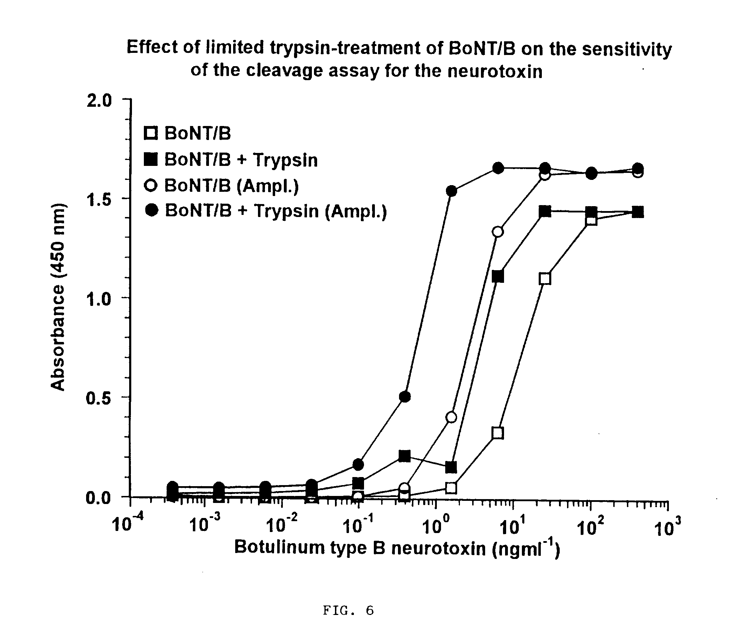

The results show that the assay of the invention can be readily used in conjunction with commercially available amplification systems to provide improved sensitivity. Figure 6 illustrates the effect of limited trypsin treatment of BoNT/B on the sensitivity of the neurotoxin assay. BoNT/B samples (1 mg/ml) were treated with trypsin (final concentration of 2.5µg/ml) for 30min at 37°C and the reaction stopped using a 5-10 molar excess of trypsin inhibitor. Figure 6 shows that, for both the amplified and non-amplified forms of the assay (see Fig.), the effect of the trypsin treatment is to increase the sensitivity of the assay system for BoNT/B.

An alternative strategy for the assay for BoNT/B is performed using a different combination of solid-phase peptide substrate and specific antibody to cleaved sequences. In this aspect of the assay the solid-phase VAMP peptide is attached by an N-terminal cysteine residue (SEQ ID NO:9, plus N-terminal cysteine):-

The specific antibody to the cleaved sequence in this embodiment is raised against the following cleaved sequence of VAMP:-

### Example 3. An Assay for Tetanus Toxin

Tetanus toxin cleaves the protein VAMP at an identical site to that of BoNT/B. The specificities of the endopeptidase activities of the two toxins however differ in the minimum peptide substrate size required for cleavage. While BoNT/B requires peptide substrates of 30-35 residues in length for optimal cleavage, the requirement for tetanus toxin is peptide substrates of > 50 residues in length.

Tetanus toxin cleavage assays are therefore carried out in an identical manner to those for BoNT/B with the exception that the solid-phase peptide is a peptide of human VAMP-1 isoform-1, residues 33-94 (SEQ ID NO:8). Example of the invention described for BoNT/B are equally applicable to tetanus toxin.

### Example 4. An Assay For Botulinum Type A Toxin (BoNT/A)

For the botulinum type A neurotoxin assay, microtitre plates are coated (10µg/ml) with the following peptide representing a sequence taken from protein SNAP-25 (aas 137-206).

Toxin test solutions buffered with 0.05M HEPES pH 7.2 containing 10µM ZnCl₂ and 10mM 2-mercaptoethanol are then added and the plate incubated at 37°C for 1h.

After washing the plates are then incubated with anti-peptide antibody conjugated to horse radish peroxidase. The antibody is specific to either of the following cleaved sequences of SNAP-25:-

After incubation the excess peptide is removed by washing and then the peroxidase substrates added to develop the colour.

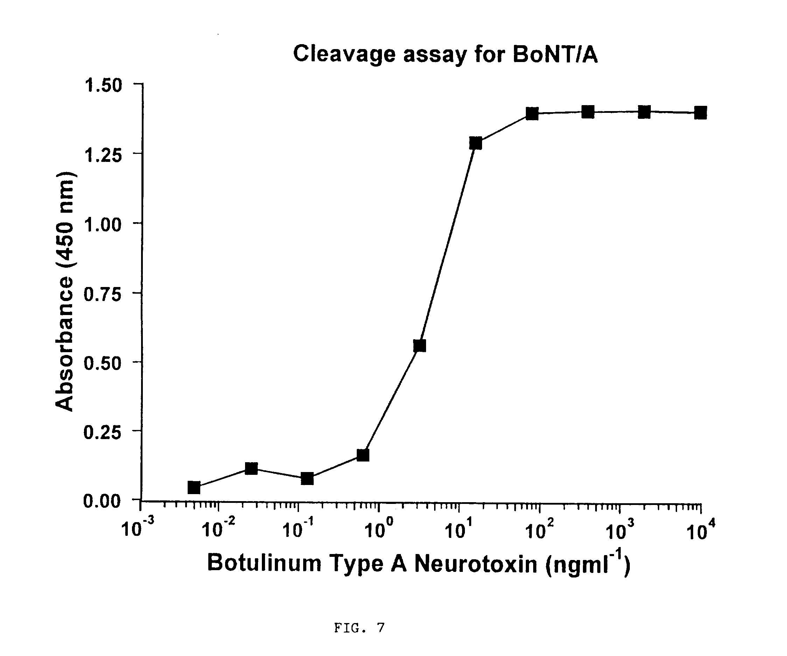

Figure 7 shows the results of a typical assay for BoNT/A. In this assays system the solid-phase peptide was SNAP-25 (137-206) as depicted above, used in conjunction with antisera specific to the cleaved sequence of SNAP-25:-

Assay protocol was as described for BoNT/B in the Example 1. The assay detected BoNT/A at a concentration of approximately 1 ng/ml (using an arbitrary absorbance cut-off point of 0.3 units above background).

The amplification systems described in Example 1 may also be applied to assays for BoNT/A.

### Example 5. An Assay Of Botulinum Type E Toxin

For the botulinum type E neurotoxin assay, microtitre plates are coated (10µg/ml) with the following peptide representing a sequence taken from protein SNAP-25 (aas 137-206).

Toxin test solutions buffered with 0.05M HEPES pH 7.2 containing 10µM ZnCl₂ and 10mM 2-mercaptoethanol are then added and the plate incubated at 37°C for 1h.

After washing the plates are then incubated with anti-peptide antibody conjugated to horse radish peroxidase. The antibody is specific to either of the following cleaved sequences of SNAP-25:-

After incubation the excess peptide is removed by washing and then the peroxidase substrates added to develop the colour.

### Example 6. Preparation And Use Of Microtitre Plates And Other Assay Solid Phases

Microtitre plate or dip-sticks are prepared by a number of different methods:-
(a) Microtitre plates are coated (100 µl/well) with the "endopeptidase peptide substrate" at a concentration of 10 or 20 µg/ml in phosphate buffered saline pH 7.4 (or water) and incubated overnight at 4°C.
(b) The "endopeptidase peptide substrate" is produced with a cysteine residue at one end. The end of the peptide to which the cysteine residue is added is determined by the specificity of the antibody to be used in the assay. The peptide cleavage product (formed during the course of the assay) which is recognised by the specific antibody must also contain the cysteine residue at its distal end. The "endopeptidase peptide substrate" is diluted to a final concentration of 10 µgml⁻¹ in 0.05 M sodium phosphate buffer, pH 6.5 containing 1mM EDTA and added (100 µl/well) to a Sulphydryl Binding Plate (Costar). After incubation for 1 hour at room temperature the peptide solution is removed and the plates washed 3 times with phosphate buffered saline , pH 7.4 (no detergent is used at this stage in the washing).
(c) A cysteine containing derivative of the "endopeptidase peptide substrate" (as above) is linked to a carrier protein (e.g. maleimide-activated BSA) which is commercially available (Pierce Warriner, U.K.) which is then bound to microtitre plates are described in (a) above.
(d) The "endopeptidase peptide substrate" is bound onto another solid-phase such as nitrocellulose membranes and procedures used similar to those defined in standard Western blotting techniques. The cleavage assay can be performed in several ways:-
   (i) The peptide substrate is cleaved on the nitrocellulose membrane.
   (ii) The peptide is cleaved by botulinum toxin in solution and then the products bound to the nitrocellulose membrane.

The plates are then washed with phosphate buffered saline (PBS) containing 0.1% Tween 20® and then blocked (to prevent further binding of proteins to the polystyrene) with a solution of 5% foetal calf serum in PBS (or 1% BSA in PBS or other blocking cocktails such as powdered milk in PBS).

Plates in this condition are suitable to be stored frozen for many months (or even years).

To perform the assay, test solutions of toxin are buffered with 0.25M Hepes pH 7.4 buffer containing 10µM ZnCl₂ and 20mM 2-dithiothreitol (toxin solution: buffer ratio of 4:1) and then added to microtitre plates and incubated for 1hr at 37°C. The toxin test solution is then washed with PBS/tween and then an antibody-peroxidase conjugate added. (The antibody is specific to one exposed end of the cleavage product and is added at a pre-determined dilution depending on the batch). After incubation for 1hr at 37°C the antibody-peroxidase conjugate is washed off the plate with PBS/tween and then peroxidase substrates added to develop the colour.

### Example 7. Method Of Specific Antibody Production

The peptides to which antibodies are to be raised are synthesised with a cysteine residue at the opposite end of interest. In Example 1, the two peptides synthesised are as follows:

The peptide is then linked to a carrier protein (e.g. Keyhole Limpet Hemocyanin or Bovine Serum Albumin) via the cysteine residue. This is achieved by mixing the peptide with the maleimide-activated carrier protein (commercially available from Pierce Warriner, U.K.) as detailed by the suppliers.

The conjugated peptide is then injected in the presence of an adjuvant (e.g. Freund's adjuvant) into animals (e.g. guinea-pigs or rabbits). One immunisation schedule is to give animals 50µg of the peptide-protein at times 0, 4 weeks and 8 weeks; animals are then bled after 10 weeks.

Antibody in serum is purified by any one of the published methods for purifying IgG from sera. Anti-peptide specific antibody is optionally further purified by affinity chromatography using the immunising peptide immobilised onto chromatography medium (kit and method commercially available from Pierce Warriner, U.K.).

Antibodies with the desired binding characteristics are assessed as describe in Example 6. The assay should not give a colour in the absence of toxin and should give a strong colour in the presence of 1µg/ml of the desired toxin.

### Example 8. Quantification Of The Assay

The assay is made quantitative by incorporating a toxin standard. The colour produced by various concentrations of the toxin is measured by a microtitre plate reader and a standard curve produced from which the toxin concentration in unknown samples may be assessed.

Thus the invention provides a novel and efficient toxin assay and provides novel reagents for use in the assay. The assay is inexpensive compared to existing assays, for example those using a mouse lethality test, and is suitable for industrial application. Further advantages of the assay and its reagents have been described above.

## Claims

1. An assay for botulinum toxin or tetanus toxin comprising the steps of:-
(a) combining a test compound with a substrate and with antibody, wherein the substrate has a cleavage site for the toxin and when cleaved by toxin forms a product, and wherein the antibody binds to the product but not to the substrate; and
(b) testing for the presence of antibody bound to the product wherein,
(i) product if present is attached to a solid phase assay component and binds the antibody; and
(ii) substrate if present is similarly attached to a solid phase assay component.

2. An assay according to Claim 1 in which the toxin is a botulinum toxin.

3. An assay according to Claim 1 or 2 comprising combining a test compound with a peptide substrate which is attached to a solid phase component of the assay.

4. An assay according to any previous claim utilizing assay components (a) and (b):
(a) a peptide linked to a solid-phase, the peptide being cleavable by the toxin to generate a cleavage product,
(b) an antibody that binds to the cleavage product but not to the peptide,
and wherein the assay comprises the steps of:
(i) combining a test compound that may contain or consist of the toxin with the solid-phase peptide to form an assay mixture,
(ii) subsequently or simultaneously combining the assay mixture with the antibody, and
(iii) subsequently or simultaneously determining whether there has been formed any conjugate between the antibody and the cleavage product.

5. An assay according to Claim 4 in which step (i) is carried out in the presence of a zinc compound.

6. An assay according to Claim 5 in which the peptide is selected from intact peptides or fragments thereof selected from VAMP, SNAP-25 and syntaxin.

7. . An assay according to Claim 6 comprising:-
(i) combining the test compound with a solid phase comprising a peptide selected from VAMP, SNAP-25, syntaxin and fragments thereof,
(ii) washing the test compound from the solid phase,
(iii) combining the solid phase with an antibody adapted for binding selectively with peptide cleaved by toxin,
(iv) detecting a conjugate of the antibody with cleaved peptide.

8. An assay according to any previous claim wherein the antibody is adapted to bind selectively to a peptide selected from SEQ ID NO:s 1-7.

9. An assay according to Claim 8 comprising:-
(i) adding a test solution to an assay plate comprising immobilized peptide, the peptide being selected from VAMP, SNAP-25, syntaxin and fragments thereof,
(ii) incubating the assay plate,
(iii) washing the plate with a buffer,
(iv) adding to the plate an antibody solution, said solution comprising an antibody adapted selectively to bind to a peptide selected from (1) a peptide the C-terminal end of which is selected from SEQ ID NO:s 1, 3 and 5, and (2) a peptide the N-terminal end of which is selected from SEQ ID NO:s 2, 4 and 6,
(v) incubating the assay plate,
(vi) washing the plate with a buffer,
(vii) measuring the presence of antibody on the assay plate.

10. An assay according to Claim 9 wherein the antibody is linked to an enzyme and the presence of antibody on the plate is measured by adding an enzyme substrate and measuring the conversion of the substrate into detectable product.

11. An assay according to Claim 10 wherein the detectable product is coloured and is measured by absorbance at a selected wavelength.

12. An assay according to any previous claim comprising converting inactive toxin present in the test compound to active toxin.

13. An assay according to Claim 12 comprising adding a protease to the test compound.

14. An assay according to any previous claim comprising detecting antibody-peptide conjugate using a further antibody specific to the first antibody and linked to an enzyme.

15. A component for a toxin assay comprising (a) a substrate for botulinum toxin or tetanus toxin, such as a peptide selected from intact peptide and fragments of VAMP, SNAP-25 and syntaxin, immobilized on (b) a solid phase, wherein said component is capable of being cleaved by botulinum toxin or tetanus toxin.

16. An assay component according to Claim 15 wherein the solid phase is selected from an assay plate, an assay well, a nitrocellulose membrane, a bead, a dipstick and a component of an elution column.

17. A toxin assay kit comprising an assay component according to Claims 15 or 16.

## Patentansprüche

1. Test auf Botulinustoxin oder Tetanustoxin, umfassend die Schritte:
(a) Vereinigen einer Testverbindung mit einem Substrat und einem Antikörper, wobei das Substrat eine Spaltstelle für das Toxin aufweist und dann, wenn es vom Toxin gespalten wird, ein Produkt bildet und worin der Antikörper das Produkt, jedoch nicht das Substrat bindet; sowie
(b) Testen auf Anwesenheit des an das Produkt gebundenen Antikörpers, wobei
(i) das Produkt, falls vorhanden, an eine Festphasentestkomponente angebunden ist und den Antikörper bindet, und
(ii) das Substrat, falls vorhanden, ebenfalls an eine Festphasentestkomponente angebunden ist.

2. Test gemäß Anspruch 1, bei dem das Toxin ein Botulinustoxin ist.

3. Test gemäß Anspruch 1 oder 2, umfassend das Vereinigen einer Testverbindung mit einem Peptidsubstrat, das an eine Festphasenkomponente des Tests gebunden ist.

4. Test gemäß einem der vorstehenden Ansprüche, welcher die Testkomponenten (a) und (b) verwendet:
(a) ein an eine Festphase gebundenes Peptid, wobei das Peptid vom Toxin zur Erzeugung eines Spaltprodukts gespalten werden kann,
(b) ein Antikörper, der das Spaltprodukt, jedoch nicht das Peptid bindet,
und worin der Test die Schritte umfaßt:
(i) Vereinigen einer Testverbindung, die das Toxin enthalten oder aus diesem bestehen kann, mit dem Festphasenpeptid zur Bildung eines Testgemischs,
(ii) anschließendes oder gleichzeitiges Vereinigen der Testmischung mit dem Antikörper, und
(iii) anschließendes oder gleichzeitiges Bestimmen, ob ein Konjugat zwischen dem Antikörper und dem Spaltprodukt gebildet wurde.

5. Test gemäß Anspruch 4, bei dem Schritt (i) in Anwesenheit einer Zinkverbindung durchgeführt wird.

6. Test gemäß Anspruch 5, bei dem das Peptid ausgewählt ist unter intakten Peptiden oder Fragmenten derselben, ausgewählt unter VAMP, SNAP-25 und Syntaxin.

7. Test gemäß Anspruch 6, umfassend:
(i) Vereinigen der Testverbindung mit einer festen Phase, umfassend ein Peptid, ausgewählt unter VAMP, SNAP-25, Syntaxin und Fragmenten derselben,
(ii) Waschen der Testverbindung von der festen Phase,
(iii) Vereinigen der festen Phasen mit einem Antikörper, der an die selektive Bindung mit von Toxin gespaltenem Peptid angepaßt ist,
(iv) Nachweisen eines Konjugats des Antikörpers mit gespaltenem Peptid.

8. Test gemäß einem der vorstehenden Ansprüche, bei dem der Antikörper an die selektive Bindung eines Peptids, ausgewählt unter den Sequenz-Nummern SEQ ID s 1-7, angepaßt ist.

9. Test gemäß Anspruch 8, umfassend:
(i) Zugeben einer Testlösung zu einer Testplatte, umfassend immobilisiertes Peptid, wobei das Peptid ausgewählt ist unter VAMP, SNAP-25, Syntaxin und Fragmenten derselben,
(ii) Inkubieren der Testplatte,
(iii) Waschen der Platte mit Puffer,
(iv) Zugeben einer Antikörperlösung zur Platte, wobei die Lösung einen Antikörper umfaßt, der an die selektive Bindung an ein Peptid angepaßt ist, das ausgewählt ist unter (1) einem Peptid, dessen C-terminales Ende ausgewählt ist unter den Sequenz-Nummern SEQ ID s 1,3 und 5, sowie (2) einem Peptid, dessen N-terminales Ende ausgewählt ist unter den Sequenz-Nummern SEQ ID s 2, 4 und 6,
(v) Inkubieren der Testplatte,
(vi) Waschen der Testplatte mit Puffer,
(vii) Messen der Anwesenheit des Antikörpers auf der Testplatte.

10. Test gemäß Anspruch 9, bei dem der Antikörper an ein Enzym gebunden ist und die Anwesenheit des Antikörpers auf der Platte durch Zugabe eines Enzymsubstrats und Messen der Umwandlung des Substrats in ein detektierbares Produkt gemessen wird.

11. Test gemäß Anspruch 10, bei dem das detektierbare Produkt gefärbt ist und mittels Absorption einer ausgewählten Wellenlänge gemessen wird.

12. Test gemäß einem der vorstehenden Ansprüche, umfassend die Umwandlung von in der Testverbindung vorhandenem inaktiven Toxin in aktives Toxin.

13. Test gemäß Anspruch 12, umfassend das Zugeben einer Protease zur Testverbindung.

14. Test gemäß einem der vorstehenden Ansprüche, umfassend die Detektion des Antikörper/Peptid-Konjugats unter Verwendung eines weiteren Antikörpers, der für den ersten Antikörper spezifisch ist und an ein Enzym gebunden ist.

15. Komponente für einen Toxintest, umfassend (a) ein Substrat für Botulinustoxin oder Tetanustoxin, wie ein Peptid, ausgewählt unter dem intakten Peptid oder Fragmenten von VAMP, SNAP-25 und Syntaxin, immobilisiert auf (b) einer Festphase, wobei die Komponente vom Botulinustoxin oder Tetanustoxin gespalten werden kann.

16. Testkomponente gemäß Anspruch 15, wobei die Festphase ausgewählt ist unter einer Testplatte, einer Testvertiefung, einer Nitrocellulosemembran, einem Kügelchen, einem Tauchstift und einer Komponente einer Elutionssäule.

17. Toxin-Testkit, umfassend eine Testkomponente gemäß einem der Ansprüche 15 oder 16.

## Revendications

1. Dosage de toxines du tétanos et du botulisme comprenant les étapes consistant à:
(a) combiner un composé de test avec un substrat et avec un anticorps, le substrat présentant un site de clivage pour la toxine et lorsqu'il a été clivé par la toxine forme un produit et l'anticorps se liant au produit et non au substrat;
(b) tester la présence d'anticorps lié au produit dans lequel,
(i) s'il est présent, le produit est fixé sur un composant de dosage en phase solide et lie l'anticorps; et
(ii) s'il est présent, le substrat est de façon similaire fixé sur un composant de dosage en phase solide.

2. Dosage selon la revendication 1, dans lequel la toxine est une toxine du botulisme.

3. Dosage selon la revendication 1 ou 2, comprenant la combinaison d'un composé de test avec un substrat peptide qui est fixé sur un composant en phase solide du dosage.

4. Dosage selon l'une quelconque des revendications précédentes faisant appel à des composants de dosage (a) et (b) :
(a) un peptide lié à une phase solide, le peptide pouvant être clivé par la toxine pour former un produit de clivage.,
(b) un anticorps qui se fixe sur le produit de clivage et non sur le peptide,
et le dosage comprenant les étapes consistant à :
(i) combiner un composé de test pouvant contenir ou consister en la toxine avec le peptide en phase solide pour former un mélange de dosage,
(ii) consécutivement ou simultanément, combiner le mélange de dosage avec l'anticorps, et
(iii) consécutivement ou simultanément, déterminer s'il a été formé un quelconque conjugué entre l'anticorps et le produit de clivage.

5. Dosage selon la revendication 4, dans lequel l'étape (i) est conduite en présence d'un composé zinc.

6. Dosage selon la revendication 5, caractérisé en ce que le peptide est choisi à partir de peptides intacts ou de leurs fragments choisis à partir de VAMP, SNAP - 25 et de syntaxine.

7. Dosage selon la revendication 6, comprenant :
(i) la combinaison du composé test avec une phase solide comprenant un peptide sélectionné à partir de VAMP, SNAP-25, syntaxine et leurs fragments,
(ii) nettoyage du composé test à partir de la phase solide,
(iii) combiner la phase solide avec un anticorps apte à se lier sélectivement avec un peptide clivé par la toxine,
(iv) détecter un conjugué de l'anticorps avec le peptide clivé.

8. Dosage selon l'une quelconque des revendications précédentes, dans lequel l'anticorps est apte à se lier sélectivement à un peptide choisi à partir de SEQ ID NO :s 1-7.

9. Dosage selon la revendication 8, comprenant les étapes consistant à :
(i)ajouter une solution test à une plaquette de dosage comprenant un peptide immobilisé, le peptide étant choisi à partir de VAMP, SNAP-25, la syntaxine et leurs fragments,
(ii) mettre la plaquette de dosage en incubation,
(iii) nettoyer la plaquette avec un tampon,
(iv) ajouter une solution anticorps à la plaquette, ladite solution comprenant un anticorps apte à se lier sélectivement sur un peptide choisi à partir de (1) un peptide dont l'extrémité terminale en C est choisie à partir de SEQ ID NO :s 1, 3 et 5, et (2) un peptide dont l'extrémité terminale en N est choisie à par partir de SEQ ID NO : 2, 4 et 6,
(v) mettre la plaquette de dosage en incubation,
(vi) nettoyer la plaquette avec un tampon,
(vii) mesurer la présence d'anticorps sur la plaquette de dosage.

10. Dosage selon la revendication 9, dans lequel l'anticorps est lié à une enzyme et la présence de l'anticorps sur la plaquette est mesurée en ajoutant un substrat enzymatique et en mesurant la conversion du substrat en produit détectable.

11. Dosage selon la revendication 10, dans lequel le produit détectable est coloré et mesuré par absorbance à une longueur d'onde sélectionnée.

12. Dosage selon l'une quelconque des revendications précédentes, comprennent la conversion de toxine inactive présente dans le composé test en toxine active.

13. Dosage selon la revendication 12, comprenant l'addition d'une protéase au composé test.

14. Dosage selon l'une quelconque des revendications précédentes, comprenant la détection d'un conjugué anticorps - peptide en utilisant un autre anticorps spécifique au premier anticorps et lié à une enzyme.

15. Composant pour un dosage de toxine, comprenant (a) un substrat pour la toxine du botulisme ou la toxine du tétanos, tel qu'un peptide choisi à partir de peptide intact et de fragments de VAMP, SNAP-25 et de syntaxine, immobilisés sur (b) une phase solide, ledit composant pouvant être clivé par la toxine de botulisme ou la toxine de tétanos.

16. Composant de dosage selon la revendication 15, dans lequel ladite phase solide est choisie à partir d'une plaquette de dosage, d'un puits de dosage, d'une membrane de nitrocellulose, d'une perle, d'une réglette - jauge et d'un composant d'une colonne d'élution.

17. Kit de dosage de toxine, comprenant un composant de dosage selon les revendications 15 ou 16.


### SEQUENCE LISTING

### (1) GENERAL INFORMATION:

(i) APPLICANT:
   (A) NAME: Microbiological Research Authority
   (B) STREET: Centre For Applied Microbiology And Research (CAMR)
   (C) CITY: Porton Down, Salisbury
   (D) STATE: Wiltshire
   (E) COUNTRY: UK
   (F) POSTAL CODE (ZIP): SP4 OJG

   (A) NAME: SHONE, Clifford Charles
   (B) STREET: 44 Oakwood Grove
   (C) CITY: Alderbury, Salisbury
   (D) STATE: Wiltshire
   (E) COUNTRY: UK
   (F) POSTAL CODE (ZIP): SP5 3BN

   (A) NAME: HALLIS, Bassam
   (B) STREET: 3 Avon Terrace
   (C) CITY: Salisbury
   (D) STATE: Wiltshire
   (E) COUNTRY: UK
   (F) POSTAL CODE (ZIP): SP2 7BT

   (A) NAME: JAMES, Benjamin, Arthur, Frederick
   (B) STREET: 22 Priory CLose
   (C) CITY: Alderbury, Salisbury
   (D) STATE: Wiltshire
   (E) COUNTRY: UK
   (F) POSTAL CODE (ZIP): SP5 3TE

   (A) NAME: QUINN, Conrad, Padraig
   (B) STREET: 36 St Fancis Road
   (C) CITY: Salisbury
   (D) STATE: Wiltshire
   (E) COUNTRY: UK
   (F) POSTAL CODE (ZIP): SP1 3QS
(ii) TITLE OF INVENTION: TOXIN ASSAY
   (iii) NUMBER OF SEQUENCES: 11
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: GB 9411138.2
      (B) FILING DATE: 30-JUN-1994

### (2) INFORMATION FOR SEQ ID NO: 1:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 1:
   Phe Glu Ser Ser Ala Ala Lys
   1               5

### (2) INFORMATION FOR SEQ ID NO: 2:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 2:
   Leu Gln Ala Gly Ala Ser Gln
   1               5

### (2) INFORMATION FOR SEQ ID NO: 3:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 3:
   Arg Ile Asp Glu Ala Asn Gln
   1               5

### (2) INFORMATION FOR SEQ ID NO: 4:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 4:
   Arg Ala Thr Lys Met Leu Gly
   1               5

### (2) INFORMATION FOR SEQ ID NO: 5:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 5:
   Gln Asn Arg Gln Ile Asp Arg
   1               5

### (2) INFORMATION FOR SEQ ID NO: 6:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 6:
   Ile Met Glu Lys Ala Asp Ser
   1               5

### (2) INFORMATION FOR SEQ ID NO: 7:

(i) SEQUENCE CHARACTERISTICS:
   (A) LENGTH: 7 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 7:
   Phe Glu Thr Ser Ala Ala Lys
   1               5

### (2) INFORMATION FOR SEQ ID NO: 8:

(i) SEQUENCE CHARACTERISTICS:
   (A) LENGTH: 62 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 8:
   Gln Gln Thr Gln Ala Gln Val Asp Glu Val Val Asp Ile Met Arg Val
   1               5                   10                  15
   Asn Val Asp Lys Val Leu Glu Arg Asp Gln Lys Leu Ser Glu Leu Asp
               20                  25                  30
   Asp Arg Ala Asp Ala Leu Gln Ala Gly Ala Ser Gln Phe Glu Ser Ser
           35                  40                  45
   Ala Ala Lys Leu Lys Arg Lys Tyr Trp Trp Lys Asn Leu Lys
       50                  55                  60

### (2) INFORMATION FOR SEQ ID NO: 9:

(i) SEQUENCE CHARACTERISTICS:
   (A) LENGTH: 35 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 9:
   Leu Ser Glu Leu Asp Asp Arg Ala Asp Ala Leu Gln Ala Gly Ala Ser
   1               5                   10                  15
   Gln Phe Glu Ser Ser Ala Ala Lys Leu Lys Arg Lys Tyr Trp Trp Lys
               20                  25                  30
   Asn Leu Lys
           35

### (2) INFORMATION FOR SEQ ID NO: 10:

(i) SEQUENCE CHARACTERISTICS:
   (A) LENGTH: 70 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 10:
   Val Thr Asn Asp Ala Arg Glu Asn Glu Met Asp Glu Asn Leu Glu Gln
   1               5                   10                  15
   Val Ser Gly Ile Ile Gly Asn Leu Arg His Met Ala Leu Asp Met Gly
               20                  25                  30
   Asn Glu Ile Asp Thr Gln Asn Arg Gln Ile Asp Arg Ile Met Glu Lys
           35                  40                  45
   Ala Asp Ser Asn Lys Thr Arg Ile Asp Glu Ala Asn Gln Arg Ala Thr
       50                  55                  60
   Lys Met Leu Gly Ser Gly
   65                  70

### (2) INFORMATION FOR SEQ ID NO: 11:

(i) SEQUENCE CHARACTERISTICS:
   (A) LENGTH: 35 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO: 11:
   Leu Ser Glu Leu Asp Asp Arg Ala Asp Ala Leu Gln Ala Gly Ala Ser
   1               5                   10                  15
   Gln Phe Glu Thr Ser Ala Ala Lys Leu Lys Arg Lys Tyr Trp Trp Lys
               20                  25                  30
   Asn Leu Lys
           35